# EUROPEAN PATENT APPLICATION

(11) **EP 4 760 262 A1**
(43) Date of publication of application: **17.06.2026**
(21) Application number: 24851616.3
(22) Date of filing: 25.07.2024
(51) Int. Cl.: G01N 35/00

(54) **METHOD FOR ADDRESSING MALFUNCTIONS IN AUTOMATIC ANALYSIS SYSTEM AND AUTOMATIC ANALYSIS DEVICE**

(30) Priority: 09.08.2023 JP 2023129750
(71) Applicant: HITACHI HIGH-TECH CORPORATION, Tokyo 105-6409 (JP)
(72) Inventor: MASHIKO, Hiroko, Tokyo 105-6409 (JP); HAGIWARA, Takaaki, Tokyo 105-6409 (JP)
(74) Representative: Strehl & Partner mbB
(86) International application number: PCT/JP2024/026578
(87) International publication number: WO 2025/033190

(57) **Abstract**

An information processing device of an automatic analysis system determines, based on operational data related to an operation of the automatic analyzer 100 stored in an operation database 32, suitability of a system parameter 31 for an actual operation of the automatic analyzer, and creates, when the system parameter is determined to be unsuitable, a recommended system parameter 33 to eliminate the unsuitability. Accordingly, it is possible to eliminate a malfunction in an automatic analyzer without a user performing a manual operation or requesting a serviceman.

## Description

### Technical Field

The present invention relates to an automatic analysis system and a malfunction-handling method for an automatic analyzer.

### Background Art

Various parameters are set in an automatic analyzer that automatically analyzes components contained in a sample such as blood or urine. The parameters are set according to a device operation, and when parameters that are not suitable for the device operation are set, alarms may occur frequently or a throughput may decrease, and original performance of a device cannot be exhibited.

PTL 1 relates to a change in parameters of an automatic analyzer. The parameters can be changed by a user administrator authority or cannot be changed without a service authority held by a serviceman of a device manufacturer. The latter parameters include parameters that may fatally affect a device operation depending on a content of change, parameters including the know-how of the device manufacturer, and the like. There is disclosed a technique in which a parameter is encoded, a code is input, and the code input in a device is decoded to change a parameter exceeding a user administrator authority without the parameter being known to a user, thereby changing a parameter requiring a service authority without waiting for a serviceman to arrive.

### Citation List

### Patent Literature

PTL 1: JP2021-169939A

### Summary of Invention

### Technical Problem

Some of the malfunctions that occur in the automatic analyzer occur because a device setting is not suitable for a situation in which the device is actually operated, and in such a case, the problem can be eliminated by changing a setting of the parameter to suit for an actual operation. In such a case, it is common for a user to change the parameter by himself/herself while viewing a handling manual, or to ask a serviceman to adjust the device.

However, as is pointed out in PTL 1, all the parameters of the device are not released to the user, and in the case of requesting the serviceman to perform adjustment, dispatch and operation of the serviceman are required, and it takes time and effort to eliminate the trouble by changing the parameters.

### Solution to Problem

An automatic analysis system according to an embodiment of the invention is an automatic analysis system including: an automatic analyzer configured to analyze a sample; and an information processing device configured to update a system parameter of the automatic analyzer. The automatic analyzer operates according to a device setting by the system parameter, the information processing device includes a processor and a memory, and when the processor loads a system parameter update program into the memory and executes the program, the information processing device determines, based on operational data related to an operation of the automatic analyzer stored in an operation database, suitability of the system parameter for an actual operation of the automatic analyzer, and creates, when the system parameter is determined to be unsuitable, a recommended system parameter to eliminate the unsuitability.

### Advantageous Effects of Invention

It is possible to eliminate a malfunction such as frequent occurrence of an alarm of an automatic analyzer without performing a manual operation of a user or requesting a serviceman. Other technical problems and novel features will become apparent from descriptions of the present description and the accompanying drawings.

### Brief Description of Drawings

[FIG. 1A] FIG. 1A is a diagram illustrating an overall configuration of an automatic analysis system.
[FIG. 1B] FIG. 1B is an example of a program and data stored in a storage device.
[FIG. 2] FIG. 2 is a system parameter update flowchart.
[FIG. 3] FIG. 3 is a data configuration example of an operation database.
[FIG. 4] FIG. 4 is a flowchart of operational data analysis and recommended system parameter creation.
[FIG. 5] FIG. 5 illustrates an example of device settings based on system parameters.
[FIG. 6] FIG. 6 is an example of recommended system parameters.
[FIG. 7] FIG. 7 is an example of an alert screen when rewriting system parameters.
[FIG. 8] FIG. 8 is an example of a system parameter rewriting confirmation screen.
[FIG. 9] FIG. 9 is an example of a machine learning model.
[FIG. 10] FIG. 10 is a flowchart of algorithm creation by the machine learning model.

### Description of Embodiments

An embodiment of the invention will be described in detail with reference to the drawings. In the following embodiment, it is needless to mention that components (also including element steps and the like) thereof are not necessarily essential unless otherwise specified or unless clearly considered to be essential in principle.

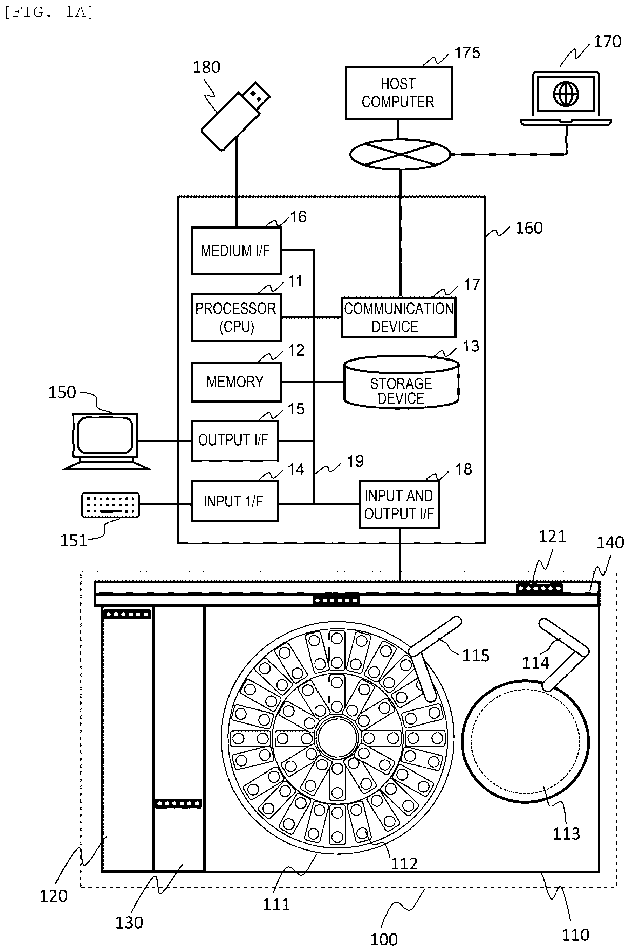

FIG. 1A is a diagram schematically illustrating an overall configuration of an automatic analysis system. The automatic analysis system includes an automatic analyzer 100 and a control device 160, and the automatic analyzer 100 includes a sample input unit 120, an analysis unit 110, a sample storage unit 130, and a transport line 140. The automatic analysis system may further include an external connection terminal 170 connected to the control device 160 via a network. The automatic analysis system is a system that provides the automatic analyzer 100 with an operation monitor function and a system parameter update function to be described later. However, in the operation monitor function and the system parameter update function, various variations are possible in the division of roles between the control device 160 and the external connection terminal 170. For example, there may be a form in which these functions are mainly carried out by the control device 160 and the external connection terminal 170 is used for backup or remote monitoring, and there may be a form in which these functions are mainly carried out by the external connection terminal 170 and the control device 160 executes update of system parameters according to an instruction from the external connection terminal 170, or a form in which these functions are separately carried out by the control device 160 and the external connection terminal 170. When the control device 160 is responsible for all of these functions, the external connection terminal 170 may be omitted.

First, a configuration of the automatic analyzer 100 will be described. The sample input unit 120 and the sample storage unit 130 are connected by the transport line 140, and the analysis unit 110 is disposed along the transport line 140. A sample container containing a sample to be analyzed is input to the sample input unit 120, and is transported to the analysis unit 110 or the sample storage unit 130 via the transport line 140. In the example, the sample container is transported in a state of being mounted on a sample rack 121 on which a plurality of sample containers can be mounted. The sample is identified by identification information such as a barcode or RFID attached to the sample container. The identification information of the sample loaded into the sample input unit 120 is read by a reading device (not illustrated) and sent to the control device 160.

The analysis unit 110 includes a reaction disk 113 that holds a reaction container, and the sample contained in the sample container transported by the transport line 140 is dispensed into the reaction container on the reaction disk 113 by a sample probe 114. On the other hand, a plurality of reagent bottles 112 containing a reagent to be used for sample analysis are held in a reagent disk 111. A reagent probe 115 dispenses a reagent in the reagent bottle 112 into the reaction container into which the sample is dispensed. The analysis unit 110 measures a mixed solution (reaction solution) of a sample and a reagent contained in a reaction container using a measuring instrument (not illustrated). For example, an analysis unit that performs biochemical analysis includes a spectroscopic analyzer and measures an absorbance, a scattered light intensity, and the like of the reaction solution. The measurement performed by the analysis unit 110 is not limited to biochemical analysis, and the analysis unit 110 includes a measuring instrument corresponding to a measurement content.

The control device 160 has a configuration as an information processing device, and includes a processor (CPU) 11, a memory 12, a storage device 13, an input interface (I/F) 14, an output interface 15, a media interface 16, a communication device 17, an input and output interface 18, and a bus 19 as main components. The processor 11 functions as a functional unit that provides a predetermined function by executing processing according to a program loaded in the memory 12. The storage device 13 stores data and a program used by the functional unit. The input I/F 14 is connected to an input device 151 such as a keyboard or a pointing device, and the output I/F 15 is connected to a display device 150. The medium I/F 16 exchanges data with a connected external medium 180. The communication device 17 enables communication with the external connection terminal 170 and a host computer 175 via a network. The external connection terminal 170 also has a configuration as an information processing device, and can exchange data with the control device 160 and remotely operate the automatic analyzer 100. The host computer 175 is a computer constituting a host system that receives an inspection to the automatic analyzer 100 and manages a result. The input and output I/F 18 communicates with each of the components included in the automatic analyzer 100. These components are communicably connected to one another via the bus 19.

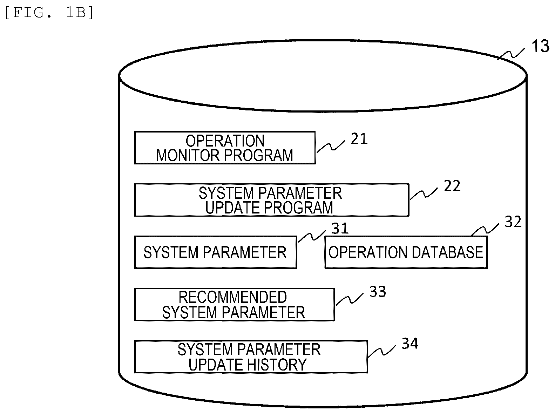

FIG. 1B illustrates an example of programs and data stored in the storage device 13. The program includes an operation monitor program 21 and a system parameter update program 22, and the data includes a system parameter 31, an operation database 32, a recommended system parameter 33, and a system parameter update history 34. Details will be described later.

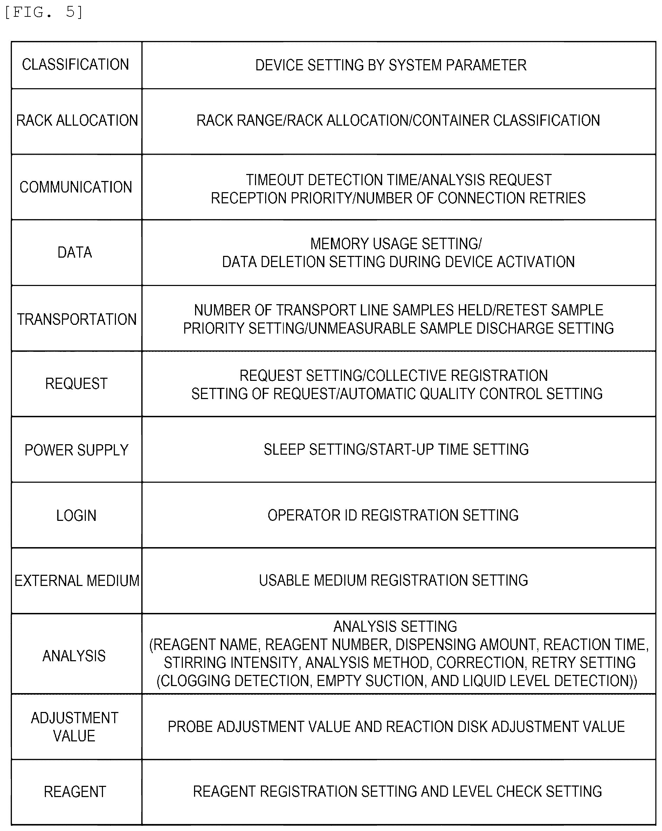

Here, the system parameter 31 is a parameter group for setting control of the automatic analyzer 100 according to an operation of the user. As illustrated in FIG. 5, there are a wide variety of device settings based on system parameters. The system parameters 31 are registered with initial values at the time of shipment of the device, and are rewritten and stored with setting values that match a device operation envisioned by the customer when the device is delivered to the customer.

As described above, when a parameter not suitable for an actual operation of the device is set as the system parameter 31, original performance of the device cannot be exhibited. In the present embodiment, in order to make it possible to troubleshoot a malfunction caused by system parameters unsuitable for an actual operation without requiring a serviceman to visit the device, the automatic analysis system monitors the actual operation of the automatic analyzer 100, creates recommended system parameters according to the actual operation, and makes it possible to update the system parameters. Accordingly, since the parameters are not updated manually, it is possible to shorten a time until the malfunction is eliminated, and it is also possible to prevent erroneous setting due to a setting error caused by a human error. Since the program is executed as a program of the system, there is no restriction of user administrator authority.

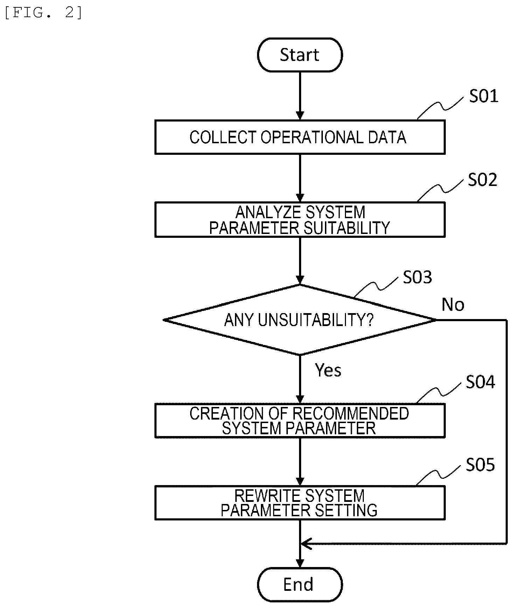

FIG. 2 illustrates a system parameter update flow according to the present embodiment. In order to execute the flow of FIG. 2, the processor 11 loads the operation monitor program 21 and the system parameter update program 22 into the memory 12 and executes the programs.

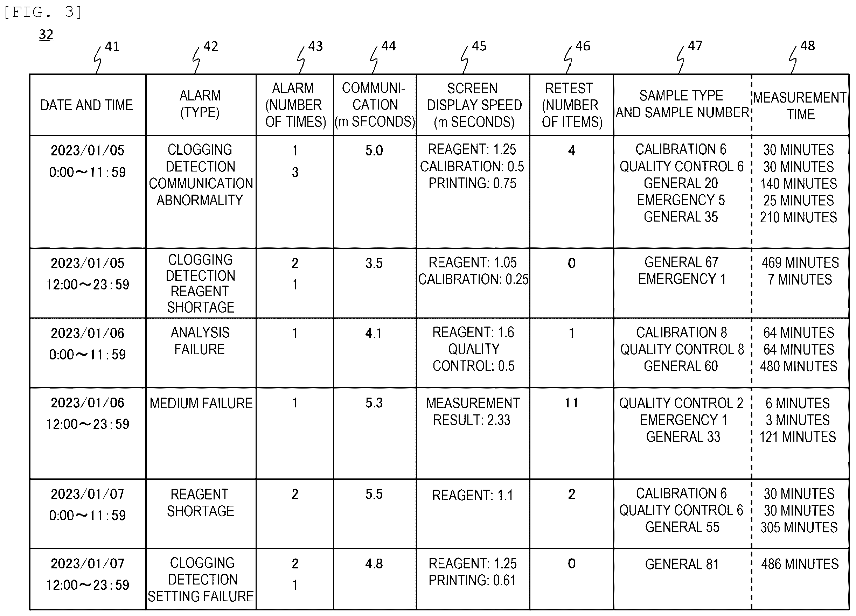

When the automatic analyzer 100 is operating, the processor 11 always executes the operation monitor program 21 and collects operational data of the automatic analyzer 100 (S01). FIG. 3 illustrates a data configuration example of the operation database 32 that stores collected operational data. Operational data is any data related to the operation of the automatic analyzer 100. Items of the operation database 32 illustrated in FIG. 3 are examples, and other items may be included, and the illustrated items are not essential. A date and time 41 indicates a date and time (time zone) when an event registered in the operation database 32 occurs. An alarm (type) 42 and an alarm (number) 43 indicate a type and a number of alarms generated at the date and time. A communication 44 indicates an average communication time between the control device 160 and the host computer 175 generated at the date and time. A screen display speed 45 indicates a processing time required for the control device 160 to display a GUI screen on the display device 150 at the date and time. For example, as an amount of data to be displayed increases, the processing time increases. A retest 46 indicates the number of items retested at the date and time. A sample type and sample number 47 and a measurement time 48 indicate the type and the number of samples measured at the date and time and the measurement time required for each sample type. The information is not limited thereto, and may include, for example, a measurement throughput, a maintenance execution situation, an input situation from the input device 151, data exchange with the external medium 180, and a communication situation with the external connection terminal 170. The operational data collected in the operation database 32 is sorted in order of date and time, for example, and is held as data indicating the state of the automatic analyzer 100 within a certain range of time.

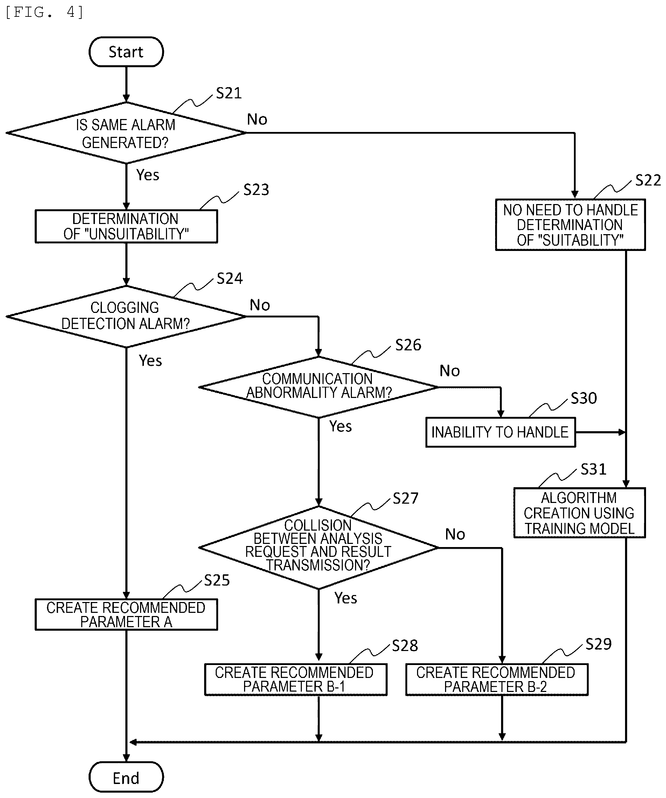

The operation database 32 is provided with a condition such as three or more times a week, for example, and periodically analyzes whether the system parameters set in the automatic analyzer 100 are suitable for an actual operation, and updates the system parameters when the system parameters are unsuitable (S02 to S05). Therefore, the control device 160 activates and executes the system parameter update program 22 at a predetermined activation timing. The system parameter update program 22 may be executed not only periodically but also at any timing by the user. Specific processing of steps S02 to S04 are illustrated in the flowchart of FIG. 4. Hereinafter, the description will be made along FIG. 4.

Whether a system parameter setting is suitable for an actual operation is determined based on an alarm generation situation in the automatic analyzer 100. The processor 11 first determines whether the same alarm occurs a predetermined number of times or more within a certain period of time using the operation database 32 (S21). When there is no same alarm that occurs beyond a threshold value, it is determined that the system parameter setting is suitable for the actual operation, and it is determined that no correspondence is required (S22). Therefore, in this case, the system parameter is not updated. On the other hand, when the same alarm is generated which exceeds the threshold value, it is determined that the system parameter setting is not suitable for the actual operation (S23), and the system parameter recommended for improving the situation is created in the following steps.

As illustrated in FIG. 5, there are a wide variety of system parameter settings, and the same alarm is frequently issued when the settings are not suitable for the actual operation. Therefore, the flow after step S24 is constructed based on a causal relationship between the system parameter and the alarm caused by the system parameter. FIG. 4 is a flowchart in which the system parameters are narrowed down and simplified, and an actual flow is constructed based on an abnormal state assumed to occur in the automatic analyzer 100 when the system parameters are unsuitable for the actual operation. That is, an abnormal state occurring in the automatic analyzer 100 is identified from the operational data of the operation database 32 by some algorithm, and a recommended system parameter acting to eliminate the abnormal state is created.

For example, frequent "clogging detection alarms" occur when the number of retries upon clogging detection is set to a normal number or a low number, even though a large amount of highly viscous reagents that are prone to clogging are used in the actual operation. Therefore, when the "clogging detection alarm" occurs frequently (S24), that is, a recommended system parameter A is created in which the number of retries at the time of detection is greater than that in normal operation (S25).

This is an example in which the recommended parameter can be created by a simple algorithm, whereas when a "communication abnormality alarm" frequently occurs (S26), some factors can be considered. For example, the factors may be due to a poor communication environment in which the device is located, a slow communication speed, or many errors in received data, or may be due to other factors. Therefore, the cause of a communication abnormality is estimated using the operation database 32. For example, it is assumed that the number of times the host computer 175 tries to transmit data to the control device 160 and the number of times the control device 160 tries to transmit data to the host computer 175 are stored in the operation database 32. When an abnormality occurs in the number of times, it can be estimated that an analysis request from the host computer 175 and the result transmission from the control device 160 collide with each other and a communication abnormality occurs. Therefore, when such an abnormality occurs (Yes in S27), a recommended system parameter B-1 that prioritizes reception of the analysis request over result transmission is created (S28).

In the example of FIG. 4, the cause of the communication abnormality is identified only in step S27 by the operation database 32, but when another cause can be identified, the cause is identified, and when it is determined that none of the causes apply, the cause is identified as being a poor communication environment for the device. In this case, the recommended system parameter B-2 in which a setting of a communication timeout detection time is longer than a timeout detection time in a normal communication environment is created (S29).

The recommended system parameter to be created may include a plurality of device settings. For example, a recommended system parameter B-3 in which the reception of the analysis request is prioritized over the result transmission and the setting of the communication timeout detection time is long can be created.

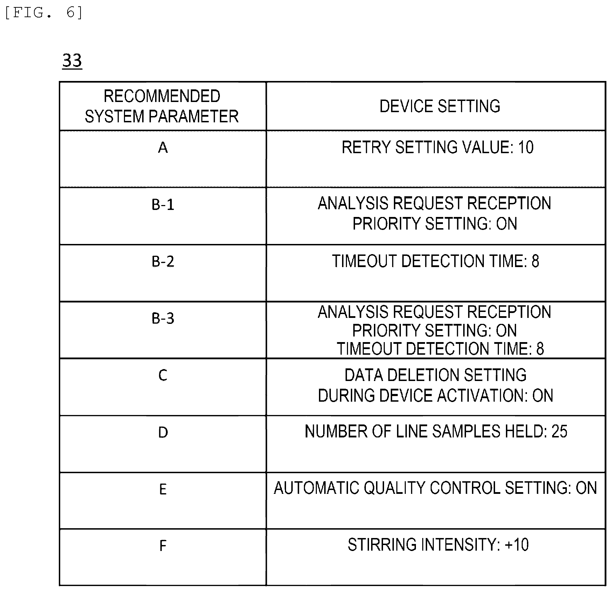

FIG. 5 illustrates device settings by system parameters for each classification. The system parameters allow for a wide variety of device settings, and in addition to using the frequent occurrence of the same alarm as a trigger, the processor 11 can detect abnormal states in the device from the operational data stored in the operation database 32 and create recommended system parameters to reduce the abnormal states. FIG. 6 illustrates the recommended system parameters.

For example, when it is determined that the screen display speed is slower than normal, a recommended system parameter C for turning on a parameter setting for deleting unnecessary data at the time of activation of the device is created.

For example, when it is determined that the device is operating in a manner that results in many analyses with shorter reaction times and faster results than normal based on the sample type and sample number and the measurement time of the measurements performed by the device, a recommended system parameter D is created that sets the number of line samples held to a large value. The recommended system parameter D has an effect of improving a measurement throughput under such an operation.

For example, when quality control analysis is always performed after calibration analysis in the operation, a recommended system parameter E for turning on automatic quality control that does not require a request for quality control by manual operation is created. The recommended system parameter E has an effect of eliminating a complicated request for quality control.

For example, when a retest rate is high and the cause of the retest is biased, a recommended system parameter F for solving the cause of the retest such as increasing a stirring intensity is created.

As described above, when the abnormality situation is identified by the determination flow of the system parameter update program 22 and the recommended system parameter for reducing the situation is created, the system parameter setting is rewritten (FIG. 2: S05).

By overwriting the currently used system parameter 31 with the recommended system parameter 33 created in the flow of FIG. 4, an abnormal state that triggers the creation of the recommended system parameter is eliminated, but another side effect may occur. Therefore, instead of simply overwriting the system parameter, it is desirable to issue to the user an alert about when updating the recommended system parameter and to leave the system parameter update history 34 so that the setting of the system parameter can be restored.

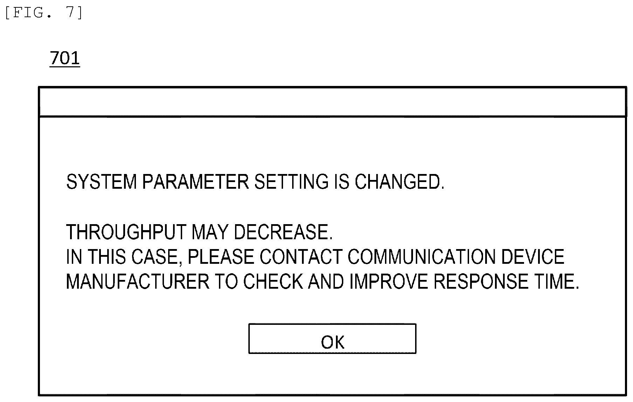

FIG. 7 illustrates an example of an alert screen displayed on the display device 150 when rewriting the system parameters. For example, on an alert screen 701, a more appropriate operation of the device is proposed by displaying a message suggesting a content of a side effect and a handling method when the side effect occurs.

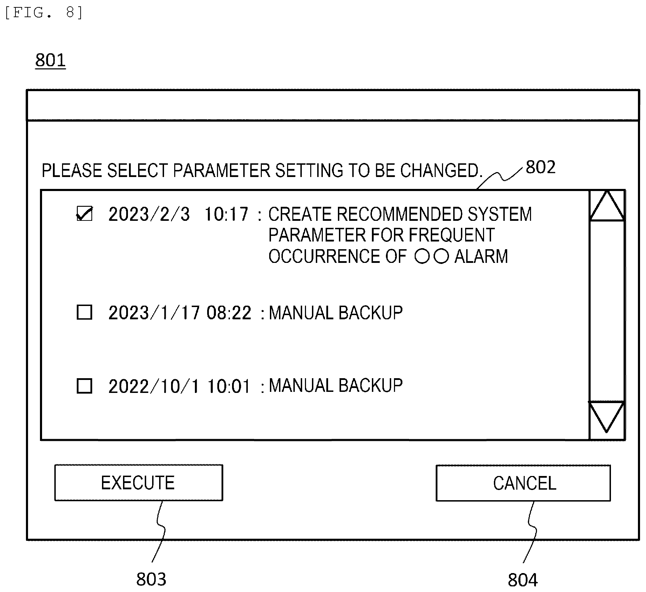

FIG. 8 illustrates a system parameter rewriting confirmation screen. A system parameter rewriting confirmation screen 801 includes a history information display unit 802, an execution button 803, and a cancel button 804. The history information display unit 802 displays system parameter update information based on the system parameter update history 34. The system parameter update history 34 stores information on a date and time of system parameter update, backup acquisition, and the like, and an updated system parameter. For example, the past system parameters themselves can be stored in the storage device 13, the external connection terminal 170, or the external medium 180, and the system parameter update history 34 can store links to these pieces of data. The user can update the system parameter by selecting a parameter setting to be changed in the history information display unit 802 and pressing the execution button 803. Since a history is displayed in the history information display unit 802, the system parameter setting can be returned to a past state by selecting a past history and pressing the execution button 803. To cancel the history selected in the history information display unit 802, the cancel button 804 is pressed.

Returning to the description of FIG. 4. Even when the same alarm frequently occurs or the abnormal state of the device can be detected based on the operational data stored in the operation database 32, an algorithm for creating a system parameter recommended for such a situation may not be able to be generated. In such a case, the system determines that the problem cannot be handled (S30), and displays a message on the display device 150, for example, prompting a serviceman to handle the problem. In the present embodiment, even in such a case, an algorithm is created by a machine learning model so that the state can be improved by updating the system parameter setting in the future (S31).

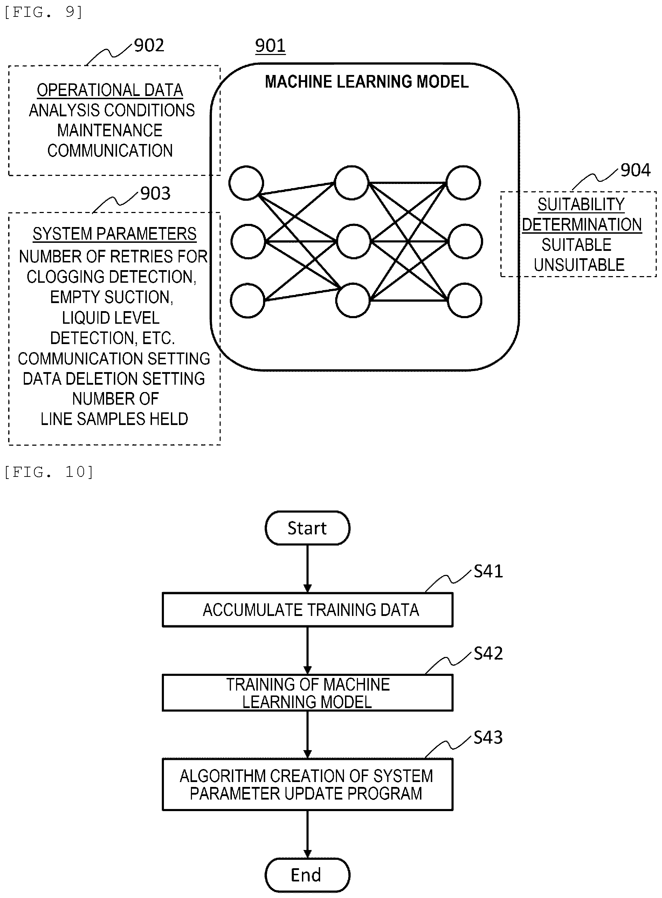

FIG. 9 illustrates an example of the machine learning model, and FIG. 10 illustrates detailed processing of step S31. Since training of a machine learning model requires a large amount of calculation resources, it is also effective to execute the training by the external connection terminal 170 instead of the control device 160. In this case, in step S31, training data to be used for training the machine learning model is created and transmitted to the external connection terminal 170 that performs training of the machine learning model.

A machine learning model 901 illustrated in FIG. 9 is a machine learning model in which, for example, an input is operational data 902 indicating an actual operation state of the automatic analyzer 100 and a system parameter 903, and an output is a suitability determination 904.

First, since the suitability of the system parameters is determined in the flow of FIG. 4, the operational data and the system parameters when the system parameters are determined to be suitable and the operational data and the system parameters when the system parameters are determined to be unsuitable are set as the training data and accumulated for training of the machine learning model 901 (S41). Thereafter, training of the machine learning model 901 is performed using the accumulated machine training data (S42). When the training is completed, it is possible to perform estimation such that suitability is determined for any system parameter under operation in a predetermined situation. Therefore, a recommended system parameter creation algorithm corresponding to the detected abnormal state is created using a trained machine learning model (S43).

The machine learning model is not limited to the model having the input and output illustrated in FIG. 9. For example, it is also possible to use a machine learning model in which the suitability determination 904 is not used, the operational data and the system parameters indicating an operation environment and an operation situation are used as inputs, the operational data indicating a malfunction situation of the device is used as an output, and a part of the operational data is estimated based on a part of the operational data and the system parameters.

Thereafter, by incorporating a creation algorithm of the recommended parameters using the trained machine learning model created in the system parameter update program 22, it is possible to create the recommended system parameters even for the abnormality situation that is not handled so far, and it is possible to expand the abnormal state that can be handled. The incorporation of the recommended parameter creation algorithm using the trained machine learning model into the program may be executed by an engineer or may be automatically added by the artificial intelligence.

The invention is not limited to the above embodiment, and includes various modifications. For example, the embodiment and the modification described above have been described in detail to facilitate understanding of the invention, and the invention is not necessarily limited to those including all the configurations described above. A part of a configuration of one embodiment or one modification can be replaced with a configuration of another embodiment or another modification, and a configuration of another embodiment or another modification can be added to a configuration of one embodiment or one modification. A part of a configuration in each of the embodiments and the modification may be added to, deleted from, or replaced with another configuration.

### Reference Signs List

- 11:: processor (CPU)
- 12:: memory
- 13:: storage device
- 14:: input interface
- 15:: output interface
- 16:: media interface
- 17:: communication device
- 18:: input and output interface
- 19:: bus
- 21:: operation monitor program
- 22:: system parameter update program
- 31:: system parameter
- 32:: operation database
- 33:: recommended system parameter
- 34:: system parameter update history
- 100:: automatic analyzer
- 110:: analysis unit
- 111:: reagent disk
- 112:: reagent bottle
- 113:: reaction disk
- 114:: sample probe
- 115:: reagent probe
- 120:: sample input unit
- 121:: sample rack
- 130:: sample storage unit
- 140:: transport line
- 150:: display device
- 151:: input device
- 160:: control device
- 170:: external connection terminal
- 175:: host computer
- 180:: external medium
- 701:: alert screen
- 801:: system parameter rewriting confirmation screen
- 802:: history information display unit
- 803:: execution button
- 804:: cancel button
- 901:: machine learning model
- 902:: operational data
- 903:: system parameter
- 904:: suitability determination

## Claims

1. An automatic analysis system comprising:
an automatic analyzer configured to analyze a sample; and
an information processing device configured to update a system parameter of the automatic analyzer, wherein
the automatic analyzer operates according to a device setting by the system parameter,
the information processing device includes a processor and a memory, and
when the processor loads a system parameter update program into the memory and executes the program, the information processing device
determines, based on operational data related to an operation of the automatic analyzer stored in an operation database, suitability of the system parameter for an actual operation of the automatic analyzer, and
creates, when the system parameter is determined to be unsuitable, a recommended system parameter to eliminate the unsuitability.

2. The automatic analysis system according to claim 1, wherein
by the processor's loading an operation monitor program into the memory and executing the program during operation of the automatic analyzer, the information processing device collects the operational data from the automatic analyzer and stores the operational data in the operation database.

3. The automatic analysis system according to claim 2, wherein
the processor periodically activates the system parameter update program.

4. The automatic analysis system according to claim 1, wherein
when the system parameter is determined to be unsuitable, the system parameter update program creates a recommended system parameter for eliminating the unsuitability by using a trained machine learning model.

5. The automatic analysis system according to claim 4, wherein
the machine learning model is a machine learning model to be trained using at least the operational data stored in the operation database and system parameters at the time of collecting the operational data as training data.

6. The automatic analysis system according to claim 1, wherein
when the information processing device rewrites a setting of the system parameter to the recommended system parameter, the information processing device issues an alert about a side effect on an operation of the automatic analyzer due to the rewriting.

7. The automatic analysis system according to claim 1, wherein
the information processing device includes a storage device,
the storage device stores a system parameter update history storing update date and time of the system parameter and information on an updated system parameter, and
the information processing device displays an update history of the system parameter on a display device based on the system parameter update history, and rewrites a setting of the system parameter to a past system parameter selected on the update history.

8. The automatic analysis system according to claim 1, wherein
the information processing device determines that the system parameter is unsuitable for an actual operation of the automatic analyzer when the automatic analyzer generates the same alarm a number of times equal to or greater than a threshold value within a predetermined period.

9. A malfunction-handling method for an automatic analyzer in an automatic analysis system including an automatic analyzer configured to analyze a sample and an information processing device configured to update a system parameter of the automatic analyzer,
the automatic analyzer operating according to a device setting by the system parameter,
the information processing device including a processor and a memory, the method comprising:
the information processing device determining, when the processor loads a system parameter update program into the memory and executes the program, based on operational data related to an operation of the automatic analyzer stored in an operation database, suitability of the system parameter for an actual operation of the automatic analyzer; and
creating, when the system parameter is determined to be unsuitable, a recommended system parameter to eliminate the unsuitability, wherein
when the automatic analyzer generates the same alarm a number of times equal to or greater than a threshold value within a predetermined period, the information processing device determines that the system parameter is unsuitable for an actual operation of the automatic analyzer.

10. The malfunction-handling method according to claim 9, wherein
by the processor's loading an operation monitor program into the memory and executing the program during operation of the automatic analyzer, the information processing device collects the operational data from the automatic analyzer and stores the operational data in the operation database.

11. The malfunction-handling method according to claim 10, wherein
the processor periodically activates the system parameter update program.

12. The malfunction-handling method according to claim 9, wherein
when the system parameter is determined to be unsuitable, the system parameter update program creates a recommended system parameter for eliminating the unsuitability by using a trained machine learning model.

13. The malfunction-handling method according to claim 12, wherein
the machine learning model is a machine learning model to be trained using at least the operational data stored in the operation database and system parameters at the time of collecting the operational data as training data.

14. The malfunction-handling method according to claim 9, wherein
when the information processing device rewrites a setting of the system parameter to the recommended system parameter, the information processing device issues an alert about a side effect on an operation of the automatic analyzer due to the rewriting.
